(19) Europäisches Patentamt
European Patent Office
Office européen des brevets

(11) Veröffentlichungsnummer : **0 108 898**
**B1**

(12) # EUROPÄISCHE PATENTSCHRIFT

(45) Veröffentlichungstag der Patentschrift : **20.08.86**

(51) Int. Cl.⁴ : **A 61 K 9/20**, A 61 K 31/505

(21) Anmeldenummer : **83109510.4**

(22) Anmeldetag : **24.09.83**

(54) **Neue orale Mopidamolformen.**

(30) Priorität : **09.10.82 DE 3237575**

(43) Veröffentlichungstag der Anmeldung : **23.05.84 Patentblatt 84/21**

(45) Bekanntmachung des Hinweises auf die Patenterteilung : **20.08.86 Patentblatt 86/34**

(84) Benannte Vertragsstaaten : **AT BE CH DE FR IT LI LU NL SE**

(56) Entgegenhaltungen :
**EP-A- 0 068 191**
**DE-A- 2 931 573**
**FR-A- 2 430 766**

(73) Patentinhaber : **Dr. Karl Thomae GmbH**
**Postfach 1755**
**D-7950 Biberach (Riss) (DE)**

(72) Erfinder : **Schepky, Gottfried, Dr.**
**Ulrich-von-Hutten-Weg 2**
**D-7950 Biberach 1 (DE)**
Erfinder : **Brickl, Rolf, Dr. Dipl.-Chem**
**Erlenweg 37**
**D-7951 Warthausen (DE)**
Erfinder : **Gruber, Peter, Dr. Dipl.-Chem.**
**Wetterkreuzstrasse 36**
**D-7950 Biberach 1 (DE)**
Erfinder : **Springmeier, Ursula**
**Benedikt-Weiser-Weg 6**
**D-7959 Mietingen 3 (DE)**
Erfinder : **Schmid, Jochen, Dr. Dipl.-Chem.**
**Panoramaweg 19**
**D-7951 Warthausen 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue orale Mopidamolformen mit einer wesentlich verbesserten Bioverfügbarkeit gegenüber der oralen Handelsform und wesentlich geringeren inter- und intraindividuellen Blutspiegelschwankungen. Diese neuen galenischen Zubereitungsformen bieten in pharmakokinetischer Hinsicht gegenüber den bisher bekannten galenischen Formen besondere Vorteile.

Das Mopidamol (2,6-Bis(diäthanolamino)-4-piperidino-pyrimido-[5,4-d]pyrimidin) stellt einen seit Jahren bekannten Wirkstoff dar. Die bisher bekannten, diesen Wirkstoff enthaltenden galenischen Zubereitungen weisen wegen der speziellen physikalischen Eigenschaften des Mopidamols für bestimmte Anwendungszwecke einige Nachteile auf.

Da Mopidamol nur im sauren Millieu gut wasserlöslich ist, kann es aus festen galenischen Formen nur dann in Lösung gehen und resorbiert werden, wenn die galenischen Zubereitungen sich ausreichend lange im sauren Bereich befinden, d. h. die Löslichkeit und damit auch die Resorption hängen stark von der Verweilzeit und dem pH-Wert im Magen und oberen Intestinaltrakt ab. Dies führt zu starken inter- und intraindividuellen Schwankungen der Blutspiegel, da Motilität des Probanden, pH-Wert des Patienten in Magen und Darm und Nahrungsaufnahme einen starken Einfluß auf die Resorption ausüben.

Aus DE-A-2 931 573 ist es bekannt Mopidamol-Wirkstoff zusammen mit Säureadditionssalzen zu verwenden, aber von einem möglichen Säureüberschuß ist nicht die Rede.

Es wurde nun eine neue, oral zu verabreichende und den Wirkstoff rasch freisetzende Mopidamol-Zubereitung gefunden, die dadurch charakterisiert ist, daß sie auf 1 Mol Mopidamol in enger Vermischung mehr als 1 Val eines oral verträglichen, sauren Hilfsstoffes, gegebenenfalls neben üblichen sonstigen Zusatzstoffen, enthält. Diese Mopidamolzubereitung zeichnet sich dadurch aus, daß sie unabhangig von den physiologischen Verhältnissen des Magen-Darmtraktes (beispielsweise unabhängig vom pH-Wert, der Pufferkapazität und der Motilität dieses Traktes) zu einer reproduzierbaren hohen Bioverfügbarkeit des Mopidamols führt.

Die erfindungsgemäßen Formen zeichnen sich dadurch aus, daß in ihnen eine innige Mischung von Mopidamol und von einem Überschuß im Verhältnis von 1 Mol Mopidamol zu mindestens 1 Val eines physiologisch unbedenklichen sauer reagierenden Stoffes vorliegt. Es war nicht zu erwarten, daß ein Überschuß an Säure zu einer signifikanten Steigerung der Bioverfügbarkeit des Mopidamols führt. Die Figur 2 zeigt an Hand der Blutspiegelwerte der oralen Handelsform, einer Filmtablette ohne Säurezusatz sowie einer solchen mit 250 mg Fumarsäurezusatz eine wesentliche Steigerung der Resorptionsgeschwindigkeit sowie resorbierten Wirkstoffmenge.

Die Abhängigkeit von Löslichkeit bzw. Auflösegeschwindigkeit des Mopidamols von der Menge an zugesetzter Säure pro Dosis wurden daraufhin untersucht ; die beiden nachstehenden Tabellen und die Figur 1 zeigen diese Abhängigkeit am Beispiel von Mopidamol-Filmtabletten mit unterschiedlichen Zusätzen an primärem Natriumcitrat bzw. Fumarsäure pro Tablette. Die Tabletten wurden *in vitro* nach der USP XX-paddle-Methode bei 100 U/min in 500 ml verdünntem Mc Ilvain-Puffer von pH 6 untersucht.

(Siehe Tabelle Seite 3 f.)

*In vitro*-Freigabegeschwindigkeit aus Mopidamol-Filmtabletten mit unterschiedlichem Fumarsäurezusatz in verdünnter Pufferlösung von pH 6 (250 mg Mopidamol/Tablette)

| | % freigesetztes Mopidamol nach Zusatz von | | | |
|---|---|---|---|---|
| nach Minuten | 0 mg | 50 mg | 125 mg | Fumarsäure/Tabletten |
| 5 | 25 | 43 | 40 | |
| 20 | 40 | 77 | 100 | |
| siehe Beispiel Nr. | 1 | 6 | 7 | |

*In vitro*-Freigabegeschwindigkeit aus Mopidamol-Filmtabletten mit unterschiedlichem Zusatz an primärem Natriumcitrat in verdünnter Pufferlösung von pH 6 (250 mg Mopidamol/Tablette)

| | % freigesetztes Mopidamol nach Zusatz von | | | | |
|---|---|---|---|---|---|
| nach Minuten | 0 mg | 25 mg | 50 mg | 100 mg | primäres Natriumcitrat |
| 5 | 25 | 30 | 35 | 48 | |
| 40 | 40 | 58 | 66 | 84 | |
| siehe Beispiel Nr. | 1 | 2 | 3 | 4 | |

Es war nicht vorhersehbar, daß Mopidamol, wenn es in nur relativ kleinen absoluten Mengen vorliegt und vollständig gelöst ist, stark übersättigte Lösungen bildet, und daß dieses Verhalten besonders dann eintritt, wenn mehr als 1 Val einer sauer reagierenden Substanz pro Mol Mopidamol vorliegt. Ein weiterer Vorteil dieser Formen besteht darin, daß infolge der pH-unabhängigen Löslichkeit das Freigabeprofil und damit auch die Blutspiegel gesteuert werden können, ohne daß ein Verlust an Bioverfügbarkeit eintritt.

Der außerordenlich überraschende Befund der Steigerung der relativen Bioverfügbarkeit gegenüber der oralen Handelsform (Mischung von Mopidamol mit Füllstoffen, wie Stärke, und Schmiermitteln, wie Magnesiumstearat) kann möglicherweise daran liegen, daß sich übersättigte, höher konzentrierte Wirkstofflösungen bilden.

Da Mopidamol eine systemisch wirkende Substanz ist, d. h. therapeutische Wirksamkeit nur besteht, solange eine möglichst vollständige Resorption aufrecht erhalten wird, ist damit eine weitere Verbesserung der therapeutischen Wirkung gegeben. Insgesamt weisen die erfindungsgemäßen neuen galenischen Formen gegenüber konventionellen Formulierungen folgende Vorteil auf :

1. Höhere Bioverfügbarkeit

2. Höhere therapeutische Sicherheit, die dadurch erreicht wird, daß einerseits die inter- und intraindividuellen Schwankungen verkleinert werden und zum anderen völlig unzureichende Blutspiegelwerte nicht auftreten.

3. Durch Steuerung der Freigabe kann eine Verbesserung der therapeutischen Wirkung und Vermeidung von Nebenwirkungen bei höheren Dosierungen erreicht werden.

4. Die höhere Bioverfügbarkeit ermöglicht in einigen Fällen eine Reduzierung der Dosis.

Es hat sich gezeigt, daß die bemerkenswerte Steigerung der relativen Bioverfügbarkeit des Mopidamols mit einer Vielzahl von Arzneiformen für die orale Anwendung realisierbar ist. Wesentlich für die hohen Blutspiegel ist die Wahl und das richtige Verhältnis von saurem Hilfsstoff zu Mopidamol.

Eingehende Untersuchungen haben gezeigt, daß mindestens 1 Val saurer Hilfsstoff für 1 Mol Mopidamol benötigt werden, um eine deutliche Verbesserung der Bioverfügbarkeit zu erreichen. Die Menge an saurem Hilfsstoff im Verhältnis zum Mopidamol ist an sich nach oben hin nicht begrenzt ; sie wird nur dadurch limitiert, daß bei zu großer Menge, keine mehr gut schluckbare orale Form des Mopidamols realisierbar ist. Bevorzugt ist ein Verhältnis von 1 bis 8 Val saurer Hilfsstoff zu 1 Mol Mopidamol. Als saure Hilsstoffe eignen sich eine ganze Reihe von pharmazeutisch unbedenklichen organischen Genußsäuren und organischen und anorganischen Salzen wie z. B. Zitronensäure, Weinsäure, Äpfelsäure, Ascorbinsäure, Fumarsäure, Bernsteinsäure, Adipinsäure usw., Monokalium- und Natriumsalze der genannten Säuren wie z. B. Monokaliumcitrat und Monokaliumtartrat aber auch Natriumhydrogensulfat, Betainhydrochlorid usw. Gelegentlich können auch Anhydride wie das Bernsteinsäureanhydrid, Glutarsäureanhydrid oder D-Glucuronsäure-γ-lacton, die in Gegenwart von Wasser zu Säuren hydrolysieren, zur Erhöhung der Löslichkeit des Mopidamols herangezogen werden. Bevorzugt werden Zubereitungsformen, die Fumarsäure enthalten. Diese Zubereitungsformen zeichnen sich durch eine hohe Lagerstabilität aus.

Die Einfachheit, mit der optimale orale feste Mopidamol-Arzneiformen realisiert werden können, war nicht vorhersehbar und für den Fachmann überaus überraschend. Im Sinne der Erfindung werden z. B. bevorzugte Mopidamol-Formen dadurch hergestellt, daß in einfacher Weise Mopidamol und Fumarsäure mit und ohne Bindemittel vermischt und dann über Tablettenpressen oder Walzenkompaktoren verdichtet werden. Die verdichteten Körper werden anschließend über Trockengranuliergeräte zur Abfüllung in Hartgelatinekapseln wieder aufgebrochen. Zur Erreichung optimaler Blutspiegel spielen die Menge der sauren Hilfsstoffe, die Art der sauren Hilfsstoffe und das Korngrößenspektrum des Trockengranulates die entscheidende Rolle.

Andererseits kann aber auch Mopidamol zusammen mit den sauren Hilfsstoffen pelletiert werden, wobei die Pellets mit einem Durchmesser von 0,1 bis 2,0 mm (vorzugsweise 0,5 bis 1,0 mm) ausgesondert und in Hartgelatinekapseln abgefüllt werden.

Die Mopidamol-Granulate und die Pellets lassen sich anschließend mit einem Lack überziehen, der im Gastrointestinaltrakt mindestens 90 % des Wirkstoffes über einen Zeitraum von 2 Stunden verteilt freigibt.

Der Wirkstoff läßt sich auch mit dem sauren Hilfsstoff sowie weiteren direkttablettierbaren Hilfsstoffen sowie dem Schmiermittel zu einer direkttablettierbaren Mischung verarbeiten, die dann zu Tablettenkernen verpreßt wird, wobei anschließend diese mit einem Lack oder geschmacksabdeckenden Überzug versehen werden. Durch weglassen des Sprengmittels und Verwendung eines verzögernden Filmüberzuges läßt sich die Wirkstoff-Freigabe über einen größerem Zeitraum hinweg verteilen (vgl. Beispiel 15).

Natürlich kann man auch zuerst den Wirkstoff mit einem bzw. mehreren sauren Hilfsstoffen zuerst feucht oder trocken granulieren, wobei nach Zumischung weiterer Hilfsstoffe die Granulate zu Tablettenkernen verpreßt werden. Man kann aber auch den Wirkstoff mit üblichen Hilfsstoffen zuerst durch Feucht- oder Trockengranulation in ein Granulat überführen, dem nachträglich die sauren Hilfsstoffe sowie ein Schmiermittel zugesetzt werden ; erst dann wird die Masse zu Tablettenkernen verpreßt.

Als besonders geeignete Säure erweist sich die Fumarsäure. Sie ist eine physiologisch völlig unbedenkliche Säure, gut verpreßbar und erzeugt in Verbindung mit Mopidamol keine hygroskopische Gemische. Erfindungswesentlich ist ihre geringe Löslichkeit ; sie sorgt dafür, daß im Gastrointestinaltrakt um das Granulatkorn stets eine ausreichende saure Mikrosphäre vorhanden ist, in der sich das schwerlösliche Mopidamol sicher und vollständig auflöst.

Wird aus medizinischen Gründen ein in seiner Höhe reduziertes, dafür aber verbreitertes Blutspiegelmaximum gewünscht, so gibt es eine ganze Reihe von galenischen Möglichkeiten. Eine Erhöhung des Säurezusatzes führt zu einer Beschleunigung der Mopidamol-Freigabe, eine Verringerung dagegen zu einer Verlangsamung der Wirkstoff-Freigabe (vgl. Figur 1).

Weitere Ausführungsformen für Mopidamol-Granulate unterscheiden sich in der Weise, daß neben gut wasserlöslichen Bindemitteln wie PVP auch in Gegenwart von Wasser schleimbildende Hilfsstoffe oder sogar wasserabstoßende Hilfsstoffe dem Mopidamol und dem sauren Hilfsstoff zugemischt werden können. Durch Zusatz von Lögungsvermittlern, wie z. B. Fettsäureglycerinpolyglykolestern, kann die Löslichkeit des Wirkstoffes sowie die Stabilität seiner Lösungen weiter verbessert werden (vgl. Beispiel 14).

Ist dagegen ein sehr rasch ansteigender Blutspiegel für das Mopidamol gewünscht, so kann man neben einer Erhöhung des Säurezusatzes oder einer Verminderung der Granulatteilchengröße (Vergrößerung der Oberfläche) besonders vorteilhaft die Art der Saüre ändern. Aufgrund der hohen Löslichkeiten insbesondere von Weinsäure, Zitronensäure, Äpfelsäure, Ascorbinsäure usw. löst sich in vitro das Mopidamol in kurzer Zeit vollständig und unabhängig vom pH-Wert des Freigabemillieus auf.

Mopidamol-Säurekombinationen lassen sich auch als Tabletten auf überraschend einfache Weise realisieren. Es zeigte sich, daß auch in Gegenwart üblicher Tablettenhilfsstoffe der Komprimiervorgang während der Tablettierung ausreicht, um eine genügend innige mechanische Verbindung zwischen Wirkstoff und zugesetzter Säure zu bewirken. Die Tablettenbeispiele mit 25, 50 und 100 mg primäres Natriumcitrat auf jeweils 250 mg Mopidamol und mit 50 und 125 mg Fumarsäure auf 250 mg Mopidamol verdeutlichen dies. Aus Geschmacksgründen wurden die Tabletten jeweils mit einem dünnen Hydroxypropyl-methylcellulose-Überzug versehen.

Ohne im einzelnen weitere Beispiele aufzuführen, ist es dem Fachmann leicht verständlich, daß durch die Art und Menge der zugesetzten Hilftsstoffe, die Art und Menge an saurem Hilfsstoff, die Art der Herstellung (Granulat-Teilchengröße) die Mopidamol-Wirkstofffreigabe in weiten Grenzen den medizinischen Anforderungen angepaßt werden kann.

So eignen sich neben den Hilfsstoffen Polyvinylpyrrolidon, hydriertes Rizinusöl und Polyacrylsäure genauso auch Hilfstoffe wie Methyl-, Äthyl-, Hydroxyäthyl- oder Hydroxypropylmethyl-cellulose. Außerdem können zur Erreichung der gewünschten Freigabe die Mischungen, bestehend aus Mopidamol und sauren Stoffen, mit in organischen Lösungsmitteln gelösten Fetten oder magensaftresistenten Lacken wie Cellulose-acetatphthalat, Schellack, Hydroxypropylmethylcellulosephthalat granuliert und dann verpreßt und wieder in Granulate gebrochen werden.

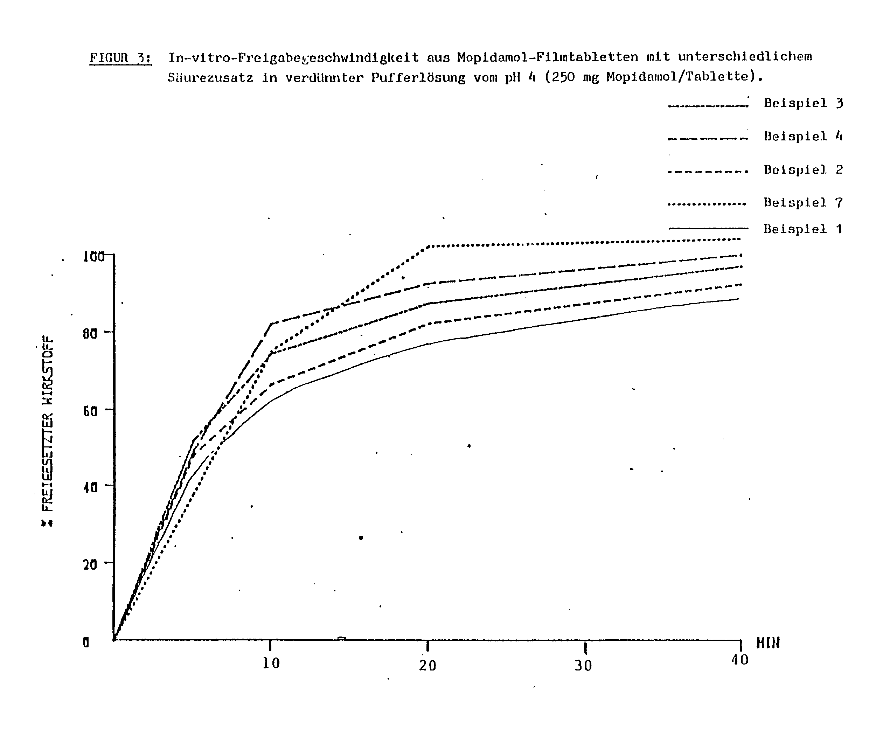

Sind aus therapeutischen Gründen höheren Mopidamol-Dosierungen notwendig und hohe Blutspiegelspitzen wegen eventueller Nebenwirkungen zu vermeiden, so gelingt dies erfindungsgemäß besonders durch die Formen, wie sie in dem Beispiel 15 beschrieben sind. Da diese Formen den Wirkstoff kontrolliert, z. B. zwischen 1 und 2,0 Stunden, abgeben, befinden sich die kleinen Teilchen daher bereits zum größten Teil im Duodenum, d. h. in einem pH-Bereich über pH 4.0 (vgl. auch Figur 3, welche die Auflösungsgeschwindigkeit bei pH 4 zeigt).

Deshalb müssen diese Formen den Wirkstoff in einen pH-Millieu freisetzen, in dem der Wirkstoff im biologischen Sinne praktisch nich mehr löslich ist. Verzögert man die Freigabe weiter, d. h. kommen die kleinen Mopidamol-Einheiten in tiefere Darmabschnitte, so ist eine vollständige Auflösung und Resorption nicht mehr gewährleistet. Das Optimum an Resorption des Mopidamols wird dadurch erreicht, daß im Sinne der Erfindung die Zusammenhänge zwischen der Art und Menge an saurem Hilfsstoff, der Art der Zuschlagstoffe, der Art der Verarbeitung, der eingestellten Wirkstofffreigabe genau bestimmt wurden.

*In vivo* Prüfung der erfindungsgemäßen Formen an Menschen :

Alle Prüfungen wurden an gesunden, freiwilligen Probanden, meist in Form von cross over Versuchen ausgeführt. Da Mopidamol nur zu einem sehr geringen Teil im Urin ausgeschieden wird, wurde als biologisch relevanter Parameter ausschließlich der Plasmaspiegel, der durch Fluoreszenzmessung bestimmt wurde, herangezogen. Es wurde verschiedene galenische Formen am Menschen überprüft. Da sich jedoch in-vivo die erfindungsgemäßen neuen, oralen und unverzögerten Formen sich gegenseitig nich signifikant unterscheiden, werden in der Figur 2 und den Tabellen 2 und 3 nur die Ergebnisse mit Beispiel 11 gezeigt.

Abbildung 2 zeigt die Mittelwerte der Mopidamol-Plasmaspiegel von 6 Probanden nach Gabe einer erfindungsgemäßen Form im Vergleich zu zwei konventionellen Formulierungen. Es zeigt sich, daß bei der säurehaltigen Form erheblich höhere Plasmaspiegel auftreten, als bei den konventionellen Formen.

Eine Vergleich der Werte der Fläche unter der Plasmaspiegelkurve (AUC) von konventionellen Tabletten oder Kapseln mit den erfindungsgemäßen Formen sowie die relativen Bioverfügbarkeiten zeigt Tabelle 2.

(Siehe Tabelle 2 Seite 6 f.)

Tabelle 2

| Probanden | AUC | | | rel. Bioverf. (%) | |
|---|---|---|---|---|---|
| | Tabletten | Kapsel | neue Form | Tab./neu | Kaps./neu |
| 1 | 0.027 | 0.073 | 0.675 | 2500 | 924 |
| 2 | 2.173 | 1,754 | 1.89 | 87 | 108 |
| 3 | 1.742 | 2.002 | 1.431 | 82 | 71 |
| 4 | 0.232 | 0.177 | 1.631 | 703 | 921 |
| 5 | 2.496 | 1.656 | 3.768 | 151 | 228 |
| 6 | 0.690 | 1.881 | 1.465 | 212 | 78 |

Die Plasmaspiegel der Einzelprobanden und Tabelle 2 zeigen, daß vor allem bei niedrigen Werten der Handelsformen und den gelegentlich auftretenden « Non-absorbern » eine sehr starke Erhöhung des Plasmaspiegels auftritt während bei Probanden die bereits mit den Handelsformen gute Plasmaspiegel aufweisen, nur eine relativ geringe Anderung eintritt. Dadurch werden die sonst üblichen Plasmaspiegelschwankungen, die eine Therapieerschwernis darstellen, deutlich verringert.

Dies zeigt auch ein Vergleich der Variationskoeffizienten von Handelsform und erfindungsgemäßer Form in Tabelle 3. Die Variationskoeffizienten der neuen Formen weisen erheblich niedrigere Werte auf.

Tabelle 3

Vergleich der Variationskoeffizienten von Handelsform auf neue form :

| Probanden | Zeit | Handelsform Tabletten | Handelsform Kapsel | neue Form |
|---|---|---|---|---|
| 1 | 0,25 | 244,7 | 69,7 | 75,8 |
| 2 | 0,50 | 155,7 | 111,9 | 47,2 |
| 3 | 0,75 | 130,8 | 97,1 | 63,0 |
| 4 | 1,00 | 96,1 | 89,0 | 55,1 |
| 5 | 1,50 | 90,3 | 96,5 | 43,4 |
| 6 | 2,00 | 97,4 | 92,9 | 44,0 |
| 7 | 3,00 | 95,6 | 84,5 | 52,2 |
| 8 | 4,00 | 83,9 | 75,7 | 60,0 |
| 9 | 5,50 | 110,8 | 67,5 | 66,5 |
| Fläche MCG × Zeit/ML | | 85,4 | 70,4 | 57,5 |

In den Zeichnungen bedeutet :
MCG/ML = Mikrogramm pro Milliliter
MG = Milligramm
Die folgenden Beispiele sollen die Erfindung näher erläutern :

Beispiel 1

Tabletten enthaltend :

| | | |
|---|---|---|
| Mopidamol | (1) | 250,0 mg |
| Milchzucker pulv. | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Triglycerid pulv. | (6) | 6,0 mg |
| Magnesiumstearat | (7) | 3,0 mg |
| | | 400,0 mg |

Herstellung von 1 000 Tabletten

1 + 2 + 3 + 4 + 5 werden mit 125 g demin. Wasser befeuchtet und durch ein Sieb mit 1,6 mm Maschenweite geschlagen. Das Granulat wird bei einer Temperatur von 45 °C im Umlufttrockenschrank getrocknet. Nochmals durch dasselbe Sieb gegeben. Anschließend werden 6 + 7 feingesiebt zugegeben, gemisch und verpreßt.

Stempeldurchmesser : 11 mm
Wölbungsradius : 11 mm

Die Tabletten werden mit Hydroxypropylmethylcellulose geschmacksabdeckend überzogen. Die fertigen Filmtabletten wiegen ca. 4 mg mehr als die Tabletten.

## Beispiel 2

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Natrium-dihydrogencitrat | (6) | 25,0 mg |
| Triglycerid pulv. | (7) | 6,0 mg |
| Magnesiumstearat | (8) | 3,0 mg |
| | | 425,0 mg |

Herstellung

1 + 2 + 3 + 4 + 5 mischen wie im Beispiel 1 angegeben. Befeuchten, Trocknen, Sieben und 6 + 7 + 8 fein-gesiebt zugeben. Mischen und pressen.

Stempeldurchmesser : 11 mm
Wölbungsradius : 11 mm

Die Tabletten werden mit Hydroxypropylmethylcellulose geschmacksabdeckend überzogen. Die fertigen Filmtabletten wiegen ca. 4,25 mg mehr als die Tabletten.

## Beispiel 3

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Natrium-dihydrogencitrat | (6) | 50,0 mg |
| Triglycerid pulv. | (7) | 6,0 mg |
| Magnesiumstearat | (8) | 3,0 mg |
| | | 450,0 mg |

Herstellung

1 + 2 + 3 + 4 + 5 mischen wie im Beispiel 1 angegeben. Befeuchten, Trocknen, Sieben und 6 + 7 + 8 feingesiebt zugeben. Mischen und pressen.

Stempeldurchmesser : 11 mm
Wölbungsradius : 11 mm

Die Tabletten werden mit Hydroxypropylmethylcellulose geschmacksabdeckend überzogen. Die fertigen Filmtabletten wiegen ca. 4,5 mg mehr als die Tabletten.

## Beispiel 4

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Natrium-dihydrogencitrat | (6) | 100,0 mg |
| Triglycerid pulv. | (7) | 6,0 mg |
| Magnesiumstearat | (8) | 3,0 mg |
| | | 500,0 mg |

Herstellung

1 + 2 + 3 + 4 + 5 mischen wie im Beispiel 1 angegeben. Befeuchten, Trocknen, Sieben und 6 + 7 + 8 feingesiebt zugeben. Mischen und pressen.
Stempeldurchmesser : 11 mm
Wölbungsradius : 11 mm
Die Tabletten werden mit Hydroxypropylmethylcellulose geschmacksabdeckend überzogen. Die fertigen Filmtabletten wiegen ca. 5,0 mg mehr als die Tabletten.

Beispiel 5

Steckkapseln enthaltend :

| | | |
|---|---|---|
| Mopidamol | (1) | 250,0 mg |
| Fumarsäure | (2) | 250,0 mg |
| Maisstärke | (3) | 37,0 mg |
| Polyvinylpyrrolidon quervernetzt | (4) | 10,0 mg |
| Milchzucker | (5) | 91,0 mg |
| Aerosil | (6) | 3,0 mg |
| Triglycerid pulv. | (7) | 6,0 mg |
| Magnesiumstearat | (8) | 3,0 mg |
| | | 650,0 mg |

Herstellung von 1 000 Steckkapseln :

1 + 2 werden gemischt und mit 50 g Aethanol befeuchtet, daraufhin durch ein Sieb mit 1,6 mm-Maschenweite geschlagen und getrocknet. Nach dem Trocknen gibt man dieses Gut nochmals durch dasselbe Sieb, 3 + 4 + 5 + 6 + 7 + 8 werden feingesiebt und zugemischt. In Gelatine-Steckkapseln der Größe 000 abfüllen.

Beispiel 6

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Fumarsäure | (6) | 50,0 mg |
| Triglycerid pulv. | (7) | 6,0 mg |
| Magnesiumstearat | (8) | 3,0 mg |
| | | 450,0 mg |

Herstellung

1 + 2 + 3 + 4 + 5 mischen wie im Beispiel 1 angegeben. Befeuchten, Trocknen, Sieben und 6 + 7 + 8 feigesiebt zugeben. Mischen und pressen.
Stempeldurchmesser : 11 mm
Wölbungsradius : 11 mm
Die Tabletten werden mit Hydroxypropylmethylcellulose geschmacksabdeckend überzogen. Die fertigen Filmtabletten wiegen ca. 4,5 mg mehr als die Tabletten.

Beispiel 7

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Fumarsäure | (6) | 125,0 mg |
| Triglycerid pulv. | (7) | 6,0 mg |
| Magnesiumstearat | (8) | 3,0 mg |
| | | 525,0 mg |

Herstellung

1 + 2 + 3 + 4 + 5 mischen wie im Beispiel 1 angegeben. Befeuchten, Trocknen, Sieben und 6 + 7 + 8 feingesiebt zugeben. Mischen und pressen.

Stempeldurchmesser : 11 mm

Wölbungsradius : 11 mm

Die Tabletten werden mit Hydroxypropylmethylcellulose geschmacksabdeckend überzogen. Die fertigen Filmtabletten wiegen ca. 5,25 mg mehr als die Tabletten.

Beispiel 8

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Weinsäure | (6) | 20,0 mg |
| Triglycerid pulv. | (7) | 6,0 mg |
| Magnesiumstearat | (8) | 3,0 mg |
| | | 420,0 mg |

Herstellung

1 + 2 + 3 + 4 + 5 mischen wie im Beispiel 1 angegeben. Befeuchten, Trocknen, Sieben und 6 + 7 + 8 feingesiebt zugeben. Mischen und pressen.

Stempeldurchmesser : 11 mm

Wölbungsradius : 11 mm

Die Tabletten werden mit Hydroxypropylmethylcellulose geschmacksabdeckend überzogen. Die fertigen Filmtabletten wiegen ca. 4,2 mg mehr als die Tabletten.

Beispiel 9

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Weinsäure | (6) | 45,0 mg |
| Triglycerid pulv. | (7) | 6,0 mg |
| Magnesiumstearat | (8) | 3,0 mg |
| | | 445,0 mg |

Herstellung

1 + 2 + 3 + 4 + 5 mischen wie im Beispiel 1 angegeben. Befeuchten, Trocknen, Sieben und 6 + 7 + 8 feingesiebt zugeben. Mischen und pressen.

Stempeldurchmesser : 11 mm

Wölbungsradius : 11 mm

Die Tabletten werden mit Hydroxypropylmethylcellulose geschmacksabdeckend überzogen. Die fertigen Filmtabletten wiegen ca. 4,5 mg mehr als die Tabletten.

Beispiel 10

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Weinsäure | (6) | 90,0 mg |
| Triglycerid pulv. | (7) | 6,0 mg |
| Magnesiumstearat | (8) | 3,0 mg |
| | | 490,0 mg |

Herstellung

1 + 2 + 3 + 4 + 5 mischen wie in Beispiel 1 angegeben. Befeuchten, Trocknen, Sieben und 6 + 7 + 8 feingesiebt zugeben. Mischen und pressen.

Stempeldurchmesser : 11 mm

Wölbungsradius : 11 mm

Die Tabletten werden mit Hydroxypropylmethylcellulose geschmacksabdeckend überzogen. Die fertigen Filmtabletten wiegen ca. 4,9 mg mehr als die Tabletten.

Beispiel 11

Tabletten enthaltend :

| | | |
|---|---|---|
| Mopidamol | (1) | 250,0 mg |
| Milchzucker | (2) | 91,0 mg |
| Maisstärke | (3) | 37,0 mg |
| Polyvinylpyrrolidon quervernetzt | (4) | 10,0 mg |
| Aerosil | (5) | 3,0 mg |
| Fumarsäure | (6) | 250,0 mg |
| Triglycerid pulv. | (7) | 6,0 mg |
| Magnesiumstearat | (8) | 3,0 mg |
| | | 650,0 mg |

Herstellung

1 + 2 + 3 + 4 + 5 mischen wie in Beispiel 1 angegeben. Befeuchten, Trocknen, Sieben und 6 + 7 + 8 feingesiebt zugeben. Mischen und pressen.

Stempeldurchmesser : 11 mm

Wölbungsradius : 11 mm

Die Tabletten werden mit Hydroxypropylmethylcellulose geschmacksabdeckend überzogen. Die fertigen Filmtabletten wiegen ca. 6,5 mg mehr als die Tabletten.

Beispiel 12

Tabletten enthaltend :

| | | |
|---|---|---|
| Mopidamol | (1) | 250,0 mg |
| Fumarsäure | (2) | 250,0 mg |
| Carbonxymethylcellulose | (3) | 97,0 mg |
| Polyvinylacetat | (4) | 20,0 mg |
| Magnesiumstearat | (5) | 3,0 mg |
| | | 620,0 mg |

Herstellung von 1 000 Filmtabletten

1 + 2 + 3 werden gemischt und mit der Lösung von 4 in 100 ml Aceton befeuchtet. Die feuchte Mischung wird durch ein Sieb mit 1,6 mm Maschenweite geschlagen, getrocknet und nochmals durch dasselbe Sieb gegeben. 5 wird zugemischt. Das fertige Granulat wird zu Tabletten verpreßt.

Stempel : 12 mm

Wölbungsradius : 10 mm

Die Tabletten werden mit Hydroxypropylmethylcellulose geschmacksabdeckend überzogen. Dadurch erhöht sich das Gewicht der Filmtabletten um ca. 6,2 mg.

Beispiel 13

Tabletten enthaltend :

| | | |
|---|---|---|
| Mopidamol | (1) | 250,0 mg |
| Weinsäure | (2) | 45,0 mg |
| Milchzucker | (3) | 91,0 mg |
| Maisstärke | (4) | 37,0 mg |
| Polyvinylpyrrolidon quervernetzt | (5) | 10,0 mg |
| Aerosil | (6) | 3,0 mg |
| Triglycerid pulv. | (7) | 6,0 mg |
| Magnesiumstearat | (8) | 3,0 mg |
| | | 445,0 mg |

Herstellung von 1 000 Filmtabletten

1 + 2 werden verrieben und mit 50 ml Aceton befeuchtet und wie im Beispiel 1 angegeben weiter bearbeitet, es werden 3 bis 8 feingesiebt zugegeben. Mischen und pressen.
Stempeldurchmesser : 11 mm
Wölbungsradius : 11 mm
Die Tabletten werden mit Hydroxypropylmethylcellulose geschmacksabdeckend überzogen. Die fertigen Filmtabletten wiegen ca. 4,5 mg mehr als die Tabletten.

Beispiel 14

Kapseln enthaltend :

| | | |
|---|---|---|
| Mopidamol | (1) | 250,0 mg |
| Fumarsäure | (2) | 250,0 mg |
| Cremophor RH 40 (R) | (3) | 200,0 mg |
| Cellulose mikrokrist | (4) | 150,0 mg |
| Kieselsäure Pyrogen | (5) | 50,0 mg |
| | | 900,0 mg |

Herstellung von 1 000 Kapseln

1 + 2 werden in 3 gut verrieben, dann wird 4 und zuletzt 5 untermischt.
Die fertige Verreibung wird in Kapseln der Größe 000 abgefüllt.

Beispiel 15

Oblong-Tabletten mit verzögerter Wirkstoff-Freigabe enthaltend :

| | |
|---|---|
| Mopidamol | 3,00 kg |
| Fumarsäure | 2,50 kg |
| Kieselsäure Pyrogen | 0,20 kg |

Diese Bestandteile werden gemischt, auf einem Walzenkompaktor verdichtet und anschließend granuliert. Nach Zumischen von 0,06 kg Magnesiumstearat wird die Mischung zu Oblong-Tabletten verpreßt. Diese werden mit einer Lösung von Methacrylsäure-Methacrylsäureestermischpolimerisat und Hydroxypropylmethyl-cellulosephthalat im Verhältniss 2 : 8 (Aceton/Isopropanol 1 : 1) besprüht, bis eine intakte Diffusionsmembran entstanden ist.

Beispiel 16

Das nach Beispiel 15 mit Hilfe eines Walzenkompators und anschließender Trockengranulation hergestellte Granulat wird mit 0,5 % Magnesiumstearat gemischt und in Hartgelatine-Steckkapseln abgefüllt.

Beispiel 17

Hartgelatine-Steckkapseln gefüllt mit Pellets folgender Zusammensetzung :

| | | |
|---|---|---|
| Weinsäure | (1) | 165,0 mg |
| Mopidamol | (2) | 200,0 mg |
| Fumarsäure | (3) | 105,0 mg |
| Polyvinylpyrrolidon | (4) | 44,0 mg |
| Talcum | (5) | 16,0 mg |
| Hydroxypropylmethylcellulose | (6) | 7,0 mg |
| | | 537,0 mg |

Sphärische Weinsäurekristalle 1 werden mit 6 überzogen. Diese werden in einem geeigneten Dragierkessel jeweils nach Befeuchten mit 4, gelöst in Isopropanol, und, in kleinen Portionen, mit einer Pulvermischung aus 2, 3 und 5 beschichtet. Nach Auftragung der gesamten Pulvermischung werden die entstandenen Pellets gesiebt, getrocknet und in Hartgelatine-Steckkapseln abgefüllt.

Beispiel 18

Hartgelatine-Steckkapsel gefüllt mit Pellets folgender Zusammensetzung :

| | | |
|---|---|---|
| Zitronensäure | (1) | 150,0 mg |
| Mopidamol | (2) | 200,0 mg |
| Fumarsäure | (3) | 105,0 mg |

| | | |
|---|---|---|
| Polyvinylpyrrolidon | (4) | 30,0 mg |
| Talcum | (5) | 16,0 mg |
| Polyoxyethylenhydrogeniertes Castor Oel | (6) | 29,0 mg |
| Hydroxypropylmethylcellulose | (7) | 7,0 mg |
| | | 537,0 mg |

Wie im Beispeil 17 beschrieben, werden die isolierten Zitronensäurekristalle mit einer Mischung aus 2, 3, 5 und 6 unter Verwendung von gelösten 4 beschichtet. Die gesiebten Pellets füllt man in Hartgelatine-Steckkapseln ab.

Beispeil 19

Pellets nach Beispiel 18 werden mit einer Lösung von Hydroxypropylmethylcellulosephthalat/Isopropanol (3 : 7) besprüht, getrocknet und in Hartgelatine-Steckkapseln abgefüllt.

**Patentansprüche** (für die Vertragsstaaten : BE CH DE FR IT LI LU NL SE)

1. Neue orale Mopidamolformen in Form von Granulaten, Pellets oder Tabletten dadurch gekennzeichnet, daß diese Formen auf 1 Mol Mopidamol (2,6-Bis(Diethanolamino)-4-piperidinopyrimido [5,4-d]-pyrimidin mehrals 1 Val eines oral verträglichen sauren Hilfsstoffes, gegebenenfalls neben üblichen Zusatzstoffen, enthalten.

2. Mopidamolformen gemäß Anspruch 1, dadurch gekennzeichnet, daß diese Formen auf 1 Mol Mopidamol 1 bis 8 Val eines oral verträglichen sauren Hilfsstoffe enthalten.

3. Mopidamolformen gemäß Anspruch 1 und 2, dadurch gekennzeichnet, daß als saure Hilfstoffe Weinsäure, Zitronensäure, Fumarsäure, Bernsteinsäure, Äpfelsäure, Ascorbinsäure, Adipinsäure, die sauren Natrium- und Kaliumsalze dieser Säuren, Natrium- oder Kaliumhydrogensulfat, Betainhydrochlorid, die in Wasser zu Säuren hydrolysierenden Anhydride der Bernsteinsäure, Glutarsäure oder das D-Glucuronsäure-4-lacton oder Gemische dieser sauren Hilfsstoffe verwendet werden.

4. Mopidamolformen gemäß den Ansprüchen 1 und 2, dadurch gekennzeichnet, daß Fumarsäure als saurer Hilfsstoff verwendet wird.

5. Mopidamolformen gemäß Anspruch 1, 2, 3 und 4, dadurch gekennzeichnet, daß das Mopidamol mit den sauren Hilfsstoffen innig vermischt in Form von Granulaten mit einer Partikelgröße zwischen 0,1 und 2,0 mm Durchmesser vorzugsweise 0,25 und 1,25 mm Durchmesser vorliegt und gegebenenfalls die Granulate, die gegebenenfalls vorher mit einem retardierenden Lack überzogen wurden, in Hartgelatinekapseln abgefüllt sind.

6. Mopidamolformen gemäß den Ansprüchen 1, 2, 3 und 4, dadurch gekennzeichnet, daß das mit den Sauren Hilfsstoffen verbundene Mopidamol in Form von Pellets mit einem Durchmesser von 0,1 bis 2,0 mm, bevorzugt 0,5 bis 1,5 mm vorliegt und gegebenenfalls die Pellets, die gegebenenfalls vorher mit einem retardierenden Lack überzogen wurden, in Hartgelatinekapseln abgefüllt sind.

7. Mopidamolformen gemäß den Ansprüchen 1 bis 5, dadurch gekennzeichnet, daß dem Mopidamol und den sauren Hilfsstoffen noch wasserlösliche Bindemittel und/oder schleimbildende Hilfsstoffe insbesondere aber Lösungsvermittler zugemischt werden.

8. Mopildamolformen gemäß Anspruch 7, dadurch gekennzeichnet, daß die Mischungen, bestehend aus Wirkstoff, sauren Hilfsstoffen, gegebenenfalls Lösungsvermittlern und anderen üblichen Hilfsstoffen in Gegenwart von Methyl-, Äthyl-, Hydroxyäthyl- oder Hydroxypropylmethylcellulose oder Polyacrylsäuren oder von Fetten verpresst und granuliert sind.

9. Mopidamolformen gemäß den Ansprüchen 1 bis 8, dadurch gekennzeichnet, daß der Wirkstoff mit einem oder mehreren sauren Hilfsstoffen sowie weiteren direkttablettierbaren Hilfsstoffen sowie dem Schmiermittel zu einer direkttablettierbaren Mischung verarbeitet ist und diese nach Verpressung zu Tablettenkernen mit einem geschmacksabdeckenden überzug versehen werden.

10. Mopidamolformen gemäß den Ansprüchen 1 bis 8, dadurch gekennzeichnet, daß der Wirkstoff mit einem bzw. mehreren sauren Hilfsstoffen durch Feucht- bzw. Trockengranulation in ein Granulat überführt wurde und dieses nach Zumischung weiterer Hilfsstoffe zu Kernen verpreßt ist und diese mit einem geschmacksabdeckenden gegebenenfalls auch den Wirkstoff langsam freigebenden Überzug versehen sind.

11. Mopidamolformen gemäß den Ansprüchen 1 bis 8, dadurch gekennzeichnet, daß der Wirkstoff mit üblichen Hilfsstoffen durch Feucht- oder Trockengranulation in ein Granulat überführt wurde und dieses nach Zumischung von einem oder mehreren sauren Hilfsstoffen sowie dem Schmiermittel zu Kernen verpreßt ist und diese mit einem geschmacksabdeckenden gegebenenfalls auch den Wirkstoff langsam freigebenden Überzug versehen sind.

12. Mopidamolformen gemäß den Anspruchen 1 bis 6 oder 9 bis 11, dadurch gekennzeichnet, daß sie einen Lacküberzug aufweisen, der im Gastrointestinaltrakt mindestens 90 % des Wirkstoffes über einen Zeitraum von 2 Stunden verteilt freigibt.

13. Mopidamolformen gemäß den Ansprüchen 1 bis 12, dadurch gekennzeichnet, daß sie als Lösungsvermittler Fettsäureglycerin-polyglykolester enthalten.

14. Mopidamolformen gemäß den Ansprüchen 1 bis 8, dadurch gekennzeichnet, daß ein Teil des sauren Hilfsstoffes mit einem Überzug aus Hydroxyäthyl- oder Hydroxypropyl-methyl-cellulose überzogen und hierauf der Wirkstoff und die übrigen Zusatzstoffe aufgetragen ist und gewünschtenfalls die dadurch entstandenen Pellets mit einem Lack überzogen und/oder die so erhaltenen Pellets gegebenenfalls in Hartgelatinekapseln abgefüllt sind.

15. Verfahren zur Herstellung von Mopidamolformen als Granulate, Pellets oder Tabletten, dadurch gekennzeichnet, daß Mopidamol und ein saurer Hilfsstoff in einem Verhältnis von 1 Mol Mopidamol (2,6-Bis(diethanolamino)-4-piperidino-pyrimido [5,4-d]-pyrimidin) zu mindestens 1 Val eines sauer reagierenden Hilfsstoffes, gegebenenfalls in Gegenwart eines Bindemittels vermischt werden, das Gemisch anschließend entweder gegebenenfalls nach Zugabe weiterer Hilfsstoffe mittels Tablettenpressen oder Walzenkompaktoren verdichtet bzw. feucht oder trocknen granuliert und wieder aufgebrochen wird, oder das Gemisch pelletiert oder direkt in Gegenwart weiterer Hilfsstoffe zu Tabletten verpreßt wird, gegebenenfalls die erhaltenen Granulate, Pellets oder Tabletten mit einem die Freigabe des Wirkstoffes regulierenden Lack oder einem geschmacksabdeckenden Überzug versehen werden und die Pellets oder Granulate gewünschtenfalls in Kapseln abgefüllt werden.

**Patentansprüche** (für den Vertragsstaat AT)

1. Verfahren zur Herstellung von Mopidamolformen als Granulate, Pellets oder Tabletten, dadurch gekennzeichnet, daß Mopidamol und ein saurer Hilfsstoff in einem Verhältnis von 1 Mol Mopidamol (2,6-Bis(diethanolamino)-4-piperidino-pyrimido [5,4-d]-pyrimidin) zu mehrals 1 Val eines sauer reagierenden Hilfsstoffes, gegebenenfalls in Gegenwart eines Bindemittels vermischt werden, das Gemisch anschließend entweder gegebenenfalls nach Zugabe weiterer Hilfsstoffe mittels Tablettenpressen oder Walzenkompaktoren verdichtet bzw. feucht oder trocknen granuliert und wieder aufgebrochen wird, oder das Gemisch pelletiert oder direkt in Gegenwart weiterer Hilfsstoffe zu Tabletten verpreßt wird, gegebenenfalls die erhaltenen Granulate, Pellets oder Tabletten mit einem die Freigabe des Wirkstoffes regulierenden Lack oder einem geschmacksabdeckenden Überzug versehen werden und die Pellets oder Granulate gewünschtenfalls in Kapseln abgefüllt werden.

2. Verfahren gemäß Anspruch 1, dadurch gekennzeichnet, daß als saure Hilfsstoffe Weinsäure, Zitronensäure, Fumarsäure, Bernsteinsäure, Äpfelsäure, Ascorbinsäure, Adipinsäure, die sauren Natrium- und Kaliumsalze dieser Säuren, Natrium- oder Kaliumhydrogensulfat, Betainhydrochlorid, die in Wasser zu Säuren hydrolysierenden Anhydride der Bernsteinsäure, Glutarsäure oder das D-Glucuronsäurelacton oder Gemische dieser sauren Hilfsstoffe verwendet werden.

3. Verfahren gemäß Anspruch 1 und 2, dadurch gekennzeichnet, daß das Mopidamol mit den sauren Hilfsstoffen innig vermischt und in die Form von Granulaten mit einer Partikelgröße zwischen 0,1 und 2,0 mm Durchmesser vorzugsweise 0,25 und 1,25 mm Durchmesser gebracht wird und gegebenenfalls die Granulate, die gegebenenfalls vorher mit einem retardierenden Lack überzogen wurden, in Hartgelatinekapseln abgefüllt werden.

4. Verfahren gemäß den Ansprüchen 1 und 2, dadurch gekennzeichnet, daß das mit den sauren Hilfsstoffen verbundene Mopidamol in die Form von Pellets mit einem Durchmesser von 0,1 bis 2,0 mm, bevorzugt 0,5 bis 1,5 mm gebracht wird und gegebenenfalls die Pellets, die Engebenenfalls vorher mit einem retardierenden Lack überzogen wurden, in Hartgelatinekapseln abgefüllt werden.

5. Verfahren gemäß den Ansprüchen 1 bis 4, dadurch gekennzeichnet, daß dem Mopidamol und den sauren Hilfsstoffen noch wasserlösliche Bindemittel und/oder schleimbildende Hilfsstoffe insbesondere aber Lösungsvermittler zugemischt werden.

6. Verfahren gemäß Anspruch 5, dadurch gekennzeichnet, daß die Mischungen, bestehend aus Wirkstoff, sauren Hilfsstoffen, gegebenenfalls Lösungsvermittlern und anderen üblichen Hilfsstoffen in Gegenwart von Methyl-, Äthyl-, Hydroxyäthyl- oder Hydroxypropylmethylcellulose oder Polyacrylsäuren oder von Fetten verpresst und granuliert werden.

7. Verfahren gemäß den Ansprüchen 1 bis 6, dadurch gekennzeichnet, daß der Wirkstoff mit einem oder mehreren sauren Hilfsstoffen sowie weiteren direkttablettierbaren Hilfsstoffen sowie dem Schmiermittel zu einer direkttablettierbaren Mischung verarbeitet wird und diese nach Verpressung zu Tablettenkernen mit einem geschmacksabdeckenden Überzug versehen werden.

8. Verfahren gemäß den Ansprüchen 1 bis 6, dadurch gekennzeichnet, daß der Wirkstoff mit einem bzw. mehreren sauren Hilfsstoffen durch Feucht- bzw. Trocknen- granulation in ein Granulat überführt und dieses nach Zumischung weiterer Hilfsstoffe zu Kernen verpreßt wird und diese mit einem geschmacksabdeckenden gegebenenfalls auch den Wirkstoff langsam freigebenden Überzug versehen werden.

9. Verfahren gemäß den Ansprüchen 1 bis 4 oder 7 und 8, dadurch gekennzeichnet, daß sie einen Lacküberzug erhalten, der im Gastrointestinaltrakt mindestens 90 % des Wirkstoffes über einen Zeitraum von 2 Stunden verteilt freigibt.

10. Verfahren gemäß den Ansprüchen 1 bis 6, dadurch gekennzeichnet, daß ein Teil des sauren Hilfsstoffes mit einem Überzug aus Hydroxyäthyl- oder Hydroxypropylmethyl-cellulose überzogen und hierauf der Wirkstoff und die übrigen Zusatzstoffe aufgetragen werden und gewünschtenfalls die dadurch entstandenen Pellets mit einem Lack überzogen und/oder die so erhaltenen Pellets gegebenenfalls in Hartgelatinekapseln abgefüllt werden.

**Claims** (for the Contracting States BE CH DE FR IT LI LU NL SE)

1. New oral mopidamol preparations in the form of granulates, pellets or tablets, characterised in that these preparations contain, for 1 mol of mopidamol (2,6-bis(diethanolamino)-4-piperidino-pyrimido [5,4-d] pyrimidine) more than 1 equivalent of an orally acceptable acidic excipient, optionally together with conventional additives.

2. Mopidamol preparations as claimed in claim 1, characterised in that these preparations contain, for 1 mol of mopidamol, from 1 to 8 equivalents of an orally acceptable acid excipient.

3. Mopidamol preparations as claimed in claims 1 and 2, characterised in that the acidic excipients used are tartaric, citric, fumaric, succinic, malic, ascorbic or adipic acid, the acid solium and potassium salts of these acids, sodium or potassium hydrogen sulphate, betaine hydrochloride, the anhydrides of succinic acid or glutaric acid which hydrolyse in water to form acids, or D-glucuronic acid-γ-lactone or mixtures of these acidic excipients.

4. Mopidamol preparations as claimed in claims 1 and 2, characterised in that fumaric acid is used as the acidic excipient.

5. Mopidamol preparations as claimed in claims 1, 2, 3 and 4 characterised in that the mopidamol, intimately mixed with the acidic excipients, is present in the form of granulates with a particle size of between 0.1 and 2.0 mm in diameter, preferably between 0.25 and 1.25 mm in diameter, and optionally the granulates, which may previously have been coated with a release-delaying coating, are packed into hard gelatine capsules.

6. Mopidamol preparations as claimed in claims 1, 2, 3 and 4 characterised in that the mopidamol combined with the acidic excipients is present in the form of pellets with a diameter of from 0.1 to 2.0 mm, preferably from 0.5 to 1.5 mm, and optionally the pellets, which may previously have been provided with a release-delaying coating, are packed into hard gelatine capsules.

7. Mopidamol preparations as claimed in claims 1 to 5, characterised in that water-soluble binders and/or mucilaginous excipients, but particulary dissolving aids, are added to the mopidamol and the acid excipients.

8. Mopidamol preparations as claimed in claim 7, characterised in that the mixtures consisting of active substance, acidic excipients, optionally dissolving aids and other conventional excipients, are compressed and granulated in the presence of methyl, ethyl, hydroxyethyl or hydroxypropylmethyl cellulose or polyacrylic acids or fats.

9. Mopidamol preparations as claimed in claims 1 to 8, characterised in that the active substance is processed with one or more acidic excipients and other excipients which are capable of being incorporated directly in tablets and with the lubricant to form a mixture capable of being made directly into tablets and after this has been compressed to form tablet cores are provided with a flavour-masking coating.

10. Mopidamol preparations as claimed in claims 1 to 8, characterised in that the active substance together with one or more acidic excipients is converted into a granulate by wet or dry granulation and after the addition of further excipients the granulate is compressed to form cores and these are then provided with a flavour-masking coating which optionally also releases the active substance slowly.

11. Mopidamol preparations as claimed in claims 1 to 8, characterised in that the active substance together with conventional excipients is converted into a granulate by wet or dry granulation and after the addition of one or more acidic excipients and the lubricant the granulate is compressed to form cores which are then provided with a flavour-masking coating which optionally also releases the active substance slowly.

12. Mopidamol preparations as claimed in claims 1 to 6 or 9 to 11,characterised in that they have a lacquer coating which, in the gastrointestinal tract, releases at least 90 % of the active substance over a period of 2 hours.

13. Mopidamol preparations as claimed in claims 1 to 12, characterised in that they contain fatty acid glycerol polyglycol esters as dissolving aids.

14. Mopidamol preparations as claimed in claims 1 to 8, characterised in that some of the acidic excipient is coated with a coating of hydroxyethyl or hydroxypropylmethyl cellulose and then the active substance and other additives are applied there to and, if desired, the resulting pellets are provided with a coating and/or the resulting pellets are optionally packed into hard gelatine capsules.

15. Process for preparing mopidamol preparations in the form of granulates, pellets or tablets, characterised in that mopidamol and an acidic excipient are mixed together in a ratio of 1 mol of mopidamol (2,6-bis(diethanolamino)-4-piperidino-pyrimido [5,4-d]-pyrimidine) to at least one equivalent of an acidically reacting excipient, optionally in the presence of a binder, then either the mixture is compressed by means of tablet presses or roller compactors, optionally after the addition of further excipients, or granulated either wet or dry and then broken up again, or the mixture is pelleted or compressed directly in the presence of other excipients to form tablets, and optionally the granulates, pellets or tablets obtained are provided with a lacquer to regulate the release of active substance or with a flavour-masking coating and the pellets or granulates are optionally packed into capsules.

**Claims** (for the Contracting State AT)

1. Process for preparing mopidamol preparations in the form of granulates, pellets or tablets, characterised in that mopidamol and an acidic excipient are mixed together in a ratio of 1 mol of mopidamol (2,6-bis(diethanolamino)-4-piperidino-pyrimido[5,4-d]-pyrimidine) to more than one equivalent of an acidically reacting excipient, optionally in the presence of a binder, then either the mixture is compressed by means of tablet presses or roller compactors, optionally after the addition of further excipients, or granulated either wet or dry and then broken up again, or the mixture is pelleted or compressed directly in the presence of other excipients to form tablets, and optionally the granulates, pellets or tablets obtained are provided with a lacquer to regulate the release of active substance or with a flavour-masking coating and the pellets or granulates are optionally packed into capsules.

2. Process as claimed in claim 1, characterised in that the acidic excipients used are tartaric, citric, fumaric, succinic, malic, ascorbic or adipic acid, the acid sodium and potassium salts of these acids, sodium or potassium hydrogen sulphate, betaine hydrochloride, the anhydrides of succinic acid or glutaric acid which hydrolyse in water to forms acids, or D-glucuronic acid-γ-lactone or mixtures of these acidic excipients.

3. Process as claimed in claims 1 and 2, characterised in that the mopidamol is intimately mixed with the acidic excipients, and made into granulates with a particle size of between 0.1 and 2.0 mm in diameter, preferably between 0.25 and 1.25 mm in diameter, and optionally the granulates, which may previously have been coated with a release-delaying coating, are packed into hard gelatine capsules.

4. Process as claimed in claims 1 and 2, characterised in that the mopidamol combined with the acidic excipients is made into pellets with a diameter of from 0.1 to 2.0 mm, preferably from 0.5 to 1.5 mm, and optionally the pellets, which have previously been provided with a release-delaying coating, are packed into hard gelatine capsules.

5. Process as claimed in claims 1 to 4, characterised in that water-soluble binders and/or mucilaginous excipients, but particularly dissolving aids, are added to the mopidamol and the acid excipients.

6. Process as claimed in claim 5, characterised in that the mixtures consisting of active substance, acidic excipients, optionally dissolving aids and other conventional excipients, are compressed and granulated in the presence of methyl, ethyl, hydroxyethyl or hydroxypropylmethyl cellulose or polyacrylic acids or fats.

7. Process as claimed in claims 1 to 6, characterised in that the active substance is processed with one or more acidic excipients and other excipients which are capable of being incorporated directly in tablets and with the lubricant to form a mixture capable of being made directly into tablets and after this has been compressed to form tablet cores the cores are provided with a flavour-masking coating.

8. Process as claimed in claims 1 to 6, characterised in that the active substance together with one or more acidic excipients is converted into a granulate by wet or dry granulation and after the addition of further excipients the granulate is compressed to form cores and these are then provided with a flavour-masking coating which optionally also releases the active substance slowly.

9. Process as claimed in claims 1 to 4 or 7 and 8, characterised in that they are given a lacquer coating which, in the gastrointestinal tract, releases at least 90 % of the active substance over a period of 2 hours.

10. Process as claimed in claims 1 to 6, characterised in that some of the acidic excipient iscoated with a coating of hydroxyethyl or hydroxypropylmethyl cellulose and then the active substance and other additives are applied thereto and, if desired, the resulting pellets are provided with a coating and/or the resulting pellets are optionally packed into hard gelatine capsules.

**Revendications** (pour les Etats contractants : BE CH DE FR IT LI LU NL SE)

1. Nouvelles formes orales de mopidamol sous forme de granulés, de pastilles ou de comprimés, caractérisées en ce que ces formes contiennent pour 1 mole de mopidamol (2,6-bis(diéthanolamino)-4-pipéridino-pyrimido[5,4-d]-pyrimidine) plus d'1 val d'un adjuvant acide compatible oralement, éventuellement en plus des additifs usuels.

2. Formes de mopidamol selon la revendication 1, caractérisées en ce que ces formes contiennent pour 1 mole de mopidamol 1 à 8 val d'un adjuvant acide compatible oralement.

3. Formes de mopidamol selon la revendication 1 et 2, caractérisées en ce que l'on utilise en tant qu'adjuvants acides, l'acide tartrique, l'acide citrique, l'acide fumarique, l'acide succinique, l'acide malique, l'acide ascorbique, l'acide adipique, les sels sodiques et potassiques acides de ces acides, le bisulfate de sodium ou de potassium, le chlorhydrate de bétaïne, les anhydrides de l'acide succinique, de l'acide glutarique s'hydrolysant en acides dans l'eau ou la γ-lactone de l'acide D-glucuronique ou des mélanges de ces adjuvants acides.

4. Formes de mopidamol selon les revendications 1 et 2, caractérisées en ce que l'on utilise l'acide fumarique en tant qu'adjuvant acide.

5. Formes de mopidamol selon la revendication 1, 2, 3 et 4, caractérisées en ce que le mopidamol intimement mélangé avec les adjuvants acides se présente sous la forme de granulés ayant une granulométrie située entre 0,1 et 2,0 mm de diamètre, de préférence entre 0,25 et 1,25 mm de diamètre et le cas échéant, les granulés qui ont éventuellement reçu au préalable un enrobage de vernis retard, sont introduits dans des capsules en gélatine dure.

6. Formes de mopidamol selon les revendications 1, 2, 3 et 4, caractérisées en ce que le mopidamol associé aux adjuvants acides se présente sous forme de pastilles ayant un diamètre de 0,1 à 2,0 mm, de préférence de 0,5 à 1,5 mm et le cas échéant, les pastilles, qui ont éventuellement reçu au préalable un enrobage de vernis retard, sont introduites dans des capsules en gélatine dure.

7. Formes de mopidamol selon les revendications 1 à 5, caractérisées en ce que l'on ajoute encore par mélange au mopidamol et aux adjuvants acides, des liants solubles dans l'eau et/ou des adjuvants formant des mucilages, en particulier toutefois, des agents de solubilisation.

8. Formes de mopidamol selon la revendication 7, caractérisées en ce que les mélanges constitués par la substance active, par les adjuvants acides, le cas échéant par des agents de solubilisation et par d'autres adjuvants usuels sont comprimés et granulés en présence de méthyl-, éthyl-, hydroxyéthyl- ou hydroxypropylméthylcellulose ou d'acides polyacryliques ou de matières grasses.

9. Formes de mopidamol selon les revendications 1 à 8, caractérisées en ce que la substance active est transformée avec un ou plusieurs adjuvants acides ainsi qu'avec d'autres adjuvants transformables directement en comprimés, de même qu'avec le lubrifiant en un mélange directement transformable en comprimés et en ce que celui-ci, après avoir été comprimé en noyaux de comprimés, est muni d'un enrobage masquant le goût.

10. Formes de mopidamol selon les revendications 1 à 8, caractérisées en ce que la substance active avec un ou plusieurs adjuvants acides a été transformée en granulés par granulation humide ou sèche et que ces granulés, après addition par mélange d'autres adjuvants, sont comprimés en noyaux et que ceux-ci sont munis d'un enrobage masquant le goût, le cas échéant également libérant lentement la substance active.

11. Formes de mopidamol selon les revendications 1 à 8, caractérisées en ce que la substance active a été transformée, avec des adjuvants usuels, en granulés par granulation humide ou sèche et que ceux-ci sont comprimés en noyaux après avoir ajouté par mélange un ou plusieurs adjuvants acides ainsi que le lubrifiant et que ces noyaux sont munis d'un enrobage masquant le goût, le cas échéant également libérant lentement la substance active.

12. Formes de mopidamol selon les revendications 1 à 6 ou 9 à 11, caractérisées en ce qu'elles présentent un enrobage en vernis, lequel libère de façon répartie, dans le tractus gastro-intestinal, au moins 90 % de la substance active dans un laps de temps de 2 heures.

13. Formes de mopidamol selon les revendications 1 à 12, caractérisées en ce qu'elles contiennent en tant qu'agent de solubilisation, des esters d'acides gras et de glycérol polyglycoliques.

14. Formes de mopidamol selon les revendications 1 à 8, caractérisées en ce qu'une partie de l'adjuvant acide est enrobée d'un enrobage constitué par de l'hydroxyéthyl- ou de l'hydroxypropylméthylcellulose sur lequel sont appliqués la substance active et les autres additifs et si on le désire, en ce que les pastilles ainsi formées sont revêtues d'un vernis et/ou les pastilles ainsi obtenues sont éventuellement introduites dans des capsules en gélatine dure.

15. Procédé pour la préparation de formes de mopidamol à l'état de granulés, pastilles ou comprimés, caractérisé en ce que l'on mélange le mopidamol et un adjuvant acide dans un rapport de 1 mole de mopidamol (2,6-bis(diéthanolamino)-4-pipéridino-pyrimido[5,4-d]-pyrimidine) pour au moins 1 val d'un adjuvant à réactivité acide, le cas échéant en présence d'un liant, on comprime ensuite le mélange, soit éventuellement après addition d'autres adjuvants au moyen de presses à comprimés ou de compacteurs à cylindres ou l'on granule par voie humide ou sèche et l'on broie de nouveau, ou on agglomère le mélange en pastilles ou on le compresse directement en présence d'autres adjuvants pour former des comprimés, le cas échéant on munit les granulés, pastilles ou comprimés obtenus d'un vernis régularisant la libération de la substance active ou d'un enrobage masquant le goût et si on le désire, on introduit les pastilles ou les granulés dans des capsules.

**Revendications** (pour l'Etat contractant AT)

1. Procédé pour la préparation de formes de mopidamol à l'état de granulés, pastilles ou comprimés, caractérisé en ce que l'on mélange le mopidamol et un adjuvant acide dans un rapport de 1 mole de mopidamol (2,6-bis(diéthanolamino)-4-pipéridino-pyrimido[5,4-d]-pyrimidine) pour au moins 1 val d'un adjuvant à réactivité acide, le cas échéant en présence d'un liant, on comprime ensuite le mélange, soit éventuellement après addition d'autres adjuvants au moyen de presses à comprimés ou de compacteurs à cylindres ou l'on granule par voie humide ou sèche et l'on broie de nouveau, ou on agglomère le mélange en pastilles ou on le compresse directement en présence d'autres adjuvants pour former des comprimés, le cas échéant on munit les granulés, pastilles ou comprimés obtenus d'un vernis régularisant la libération de la substance active ou d'un enrobage masquant le goût et si on le désire, on introduit les pastilles ou les granulés dans des capsules.

2. Procédé selon la revendication 1, caractérisé en ce que l'on utilise en tant qu'adjuvants acides, l'acide tartrique, l'acide citrique, l'acide fumarique, l'acide succinique, l'acide malique, l'acide ascorbique, l'acide adipique, les sels sodiques et potassiques acides de ces acides, le bisulfate de sodium ou de potassium, le chlorhydrate de bétaïne, les anhydrides de l'acide succinique, de l'acide glutarique s'hydrolysant en acides dans l'eau ou la lactone de l'acide D-glucuronique ou des mélanges de ces adjuvants acides.

3. Procédé selon la revendication 1 et 2, caractérisé en ce que l'on mélange intimement le mopidamol avec les adjuvants acides et qu'on l'amène sous la forme de granulés ayant une granulométrie située entre 0,1 et 2,0 mm de diamètre, de préférence entre 0,25 et 1,25 mm de diamètre et le cas échéant, on introduit les granulés qui ont éventuellement reçu au préalable un enrobage de vernis retard, dans des capsules en gélatine dure.

4. Procédé selon les revendications 1 et 2, caractérisé en ce que l'on amène le mopidamol associé aux adjuvants acides sous la forme de pastilles ayant un diamètre de 0,1 à 0,2 mm, de préférence de 0,5 à 1,5 mm et le cas échéant, on introduit les pastilles, qui ont éventuellement reçu au préalable un enrobage de vernis retard dans des capsules en gélatine dure.

5. Procédé selon les revendications 1 à 4, caractérisé en ce que l'on ajoute en outre par mélange au mopidamol et aux adjuvants acides des liants solubles dans l'eau et/ou des adjuvants formant des mucilages, en particulier toutefois, des agents de solubilisation.

6. Procédé selon la revendication 5, caractérisé en ce que l'on comprime et l'on granule les mélanges constitués par la substance active, par les adjuvants acides, le cas échéant par des agents de solubilisation et par d'autres adjuvants usuels en présence de méthyl-, éthyl-, hydroxyéthyl- ou hydroxypropylméthylcellulose ou d'acides polyacryliques ou de graisses.

7. Procédé selon les revendications 1 à 6, caractérisé en ce que l'on traite la substance active avec un ou plusieurs adjuvants acides ainsi qu'avec d'autres adjuvants transformables directement en comprimés de même qu'avec le lubrifiant pour obtenir un mélange directement transformable en comprimés, et en ce que l'on munit celui-ci, après l'avoir comprimé en noyaux de comprimés d'un enrobage masquant le goût.

8. Procédé selon les revendications 1 à 6, caractérisé en ce que la substance active avec un ou plusieurs adjuvants acides est transformée en granulés par granulation humide ou sèche et ces granulés, après addition par mélange d'autres adjuvants, sont comprimés en noyaux et ceux-ci sont munis d'un enrobage masquant le goût, le cas échéant également libérant lentement de la substance active.

9. Procédé selon les revendications 1 à 4 ou 7 et 8, caractérisé en ce qu'ils présentent un enrobage en vernis, lequel libère de façon répartie, dans le tractus gastro-intestinal, au moins 90 % de la substance active dans un laps de temps de 2 heures.

10. Procédé selon les revendications 1 à 6, caractérisé en ce qu'une partie de l'adjuvant acide est enrobée d'un enrobage constitué par de l'hydroxyéthyl- ou de l'hydroxypropylméthylcellulose sur lequel on applique la substance active et les autres additifs et si on le désire, on revêt les pastilles ainsi formées d'un vernis et/ou on introduit les pastilles ainsi obtenues éventuellement dans des capsules en gélatine dure.

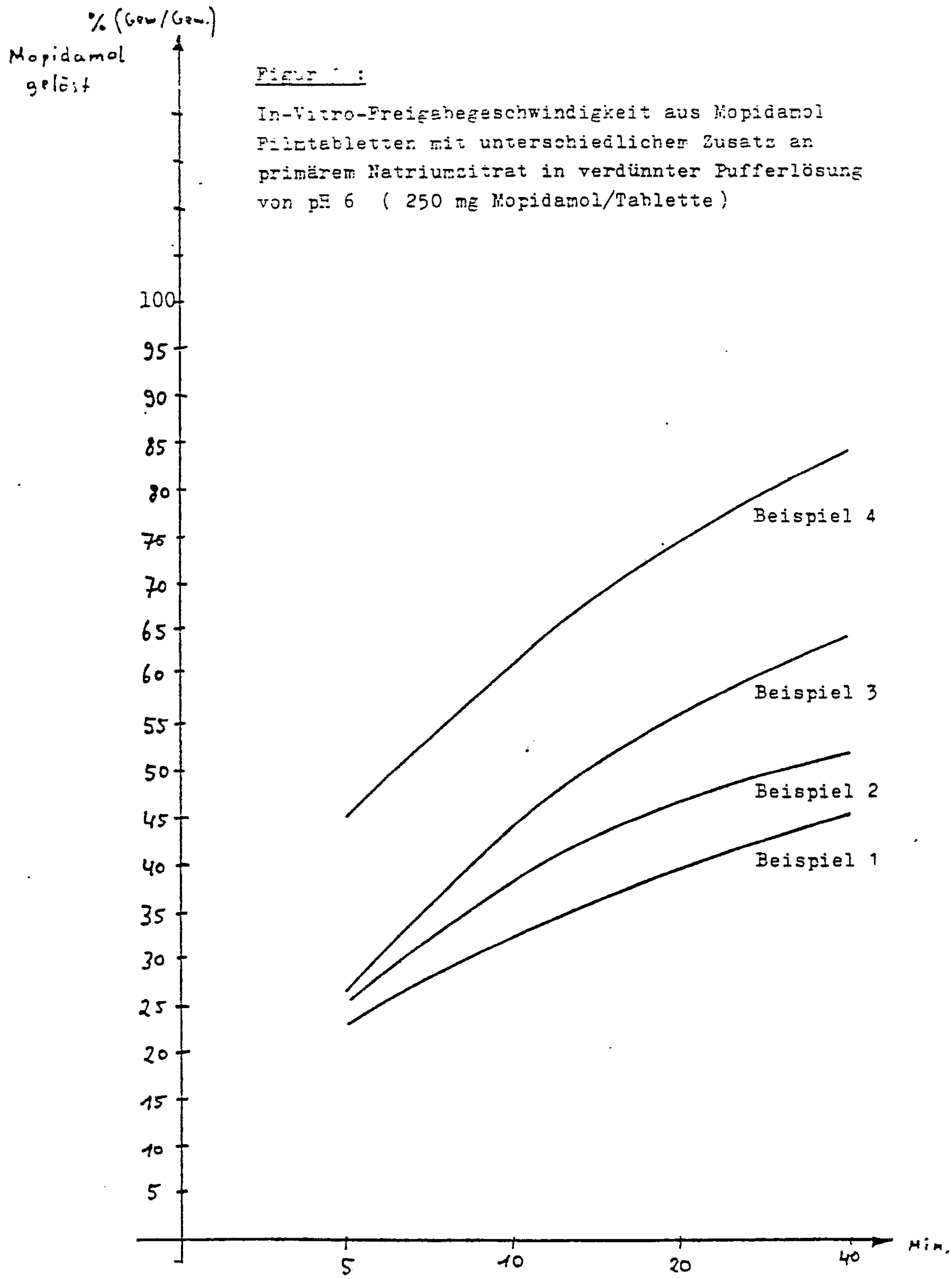


Figur 1 :

In-Vitro-Freigabegeschwindigkeit aus Mopidamol Filmtabletten mit unterschiedlichem Zusatz an primärem Natriumzitrat in verdünnter Pufferlösung von pH 6 ( 250 mg Mopidamol/Tablette )

Figur 2:

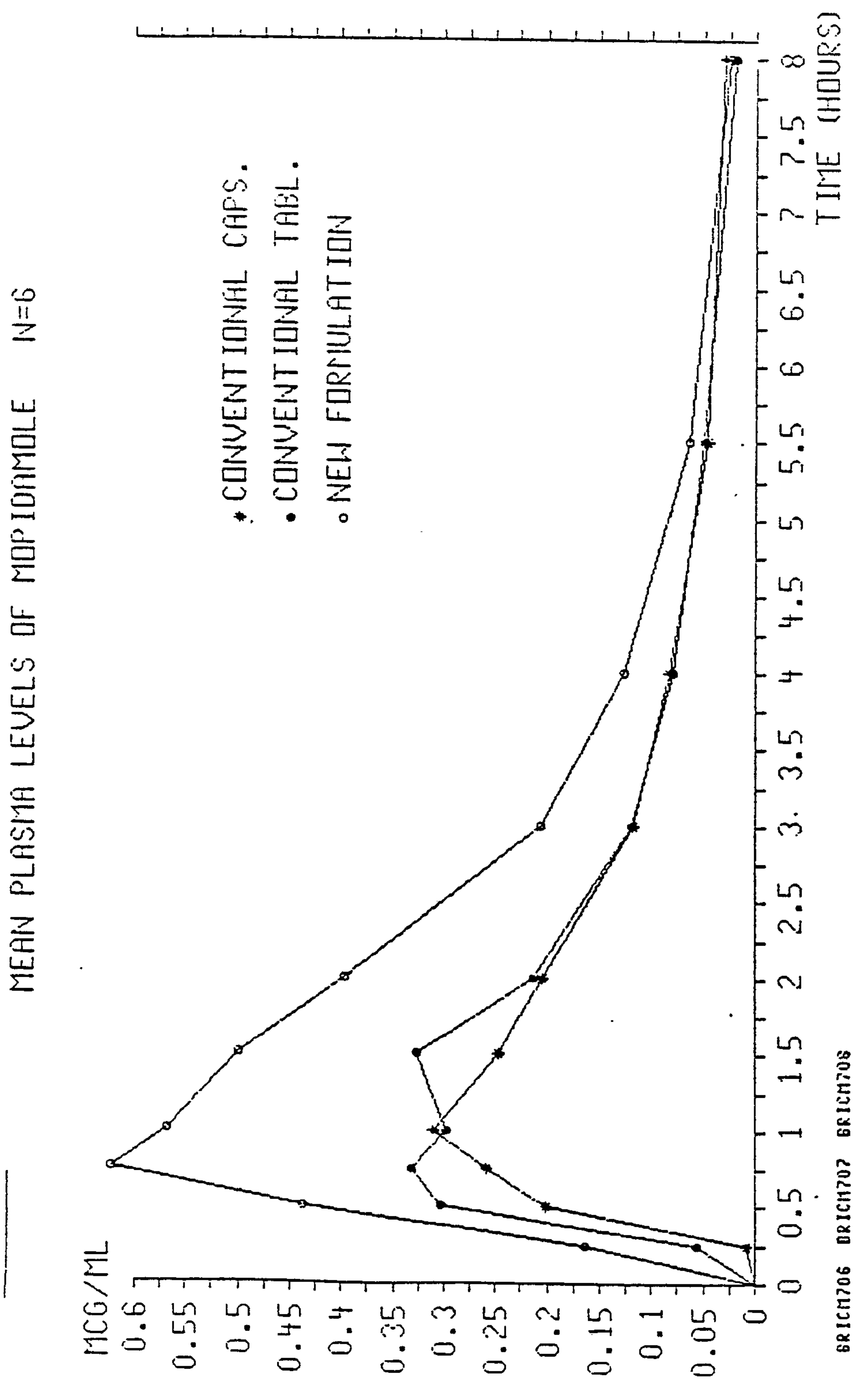
MEAN PLASMA LEVELS OF MOPIDAMOLE N=6
* CONVENTIONAL CAPS.
• CONVENTIONAL TABL.
○ NEW FORMULATION
MCG/ML
0.6
0.55
0.5
0.45
0.4
0.35
0.3
0.25
0.2
0.15
0.1
0.05
0
0 0.5 1 1.5 2 2.5 3. 3.5 4 4.5 5 5.5 6 6.5 7 7.5 8
TIME (HOURS)
GRICH706 GRICH707 GRICH708
AUSDRUCK VOM 00.10.82/KU

FIGUR 3: In-vitro-Freigabegeschwindigkeit aus Mopidamol-Filmtabletten mit unterschiedlichem Säurezusatz in verdünnter Pufferlösung vom pH 4 (250 mg Mopidamol/Tablette).